# EUROPEAN PATENT SPECIFICATION

(11) **EP 3 721 788 B1**
(45) Date of publication and mention of the grant of the patent: **10.09.2025**
(21) Application number: 18885428.5
(22) Date of filing: 28.11.2018
(51) Int. Cl.: A61B 3/103, A61B 3/10, A61B 3/028, A61B 3/00, A61B 3/12

(54) **OCULAR REFRACTIVE POWER MEASUREMENT DEVICE**
VORRICHTUNG ZUR MESSUNG DER OKULAREN BRECHKRAFT
DISPOSITIF DE MESURE DE PUISSANCE RÉFRACTIVE OCULAIRE

(30) Priority: 04.12.2017 JP 2017232328
(43) Date of publication of application: 14.10.2020
(73) Proprietor: Nidek Co., Ltd., Gamagori-shi, Aichi 443-0038 (JP)
(72) Inventor: TAKII, Michihiro, Gamagori-shi Aichi 443-0038 (JP); HANEBUCHI, Masaaki, Gamagori-shi Aichi 443-0038 (JP); OCHI, Hisashi, Gamagori-shi Aichi 443-0038 (JP)
(74) Representative: Stöckeler, Ferdinand
(86) International application number: PCT/JP2018/043845
(87) International publication number: WO 2019/111788

(56) References cited:
- EP-A1- 2 106 741
- EP-B1- 2 106 741
- WO-A1-2016/073887
- WO-A1-2017/002846
- JP-A- 2001 161 644
- JP-A- 2001 161 644
- JP-A- 2014 045 924
- JP-A- H02 156 927
- JP-A- H02 156 927
- JP-A- H09 253 049
- JP-A- H09 253 049
- US-A1- 2015 272 436
- US-A1- 2015 374 224

## Description

### TECHNICAL FIELD

The present disclosure relates to an eye refractive power measurement apparatus that measures the eye refractive power of an examinee's eye.

### BACKGROUND ART

An apparatus that is assumed to measure the eye refractive power in an eyeglasses worn state has been proposed as an eye refractive power apparatus that measures the eye refractive power of an examinee's eye (refer to, for example, Patent Literature 1).

EP 2 106 741 A1 discloses a fundus camera in which an alignment target projection optical system for projecting alignment target light is configured such that a plurality of infrared light sources are concentrically arranged at intervals of 45 degrees having an optical axis at the center. JP 2001 161644 A describes an eye refractive power measurement apparatus comprising: a measuring optical system for projecting measurement light onto the fundus of an examinee's eye, receiving reflected light therefrom, and accordingly objectively measuring the eye refractive power of the examinee's eye; an illuminating light source for illuminating the anterior segment of the examinee's eye in an eyeglasses worn state; and an observing optical system configured to observe the anterior segment of the examinee's eye in the eyeglasses worn state, the anterior segment being illuminated by the illuminating light source, wherein the eye refractive power measurement apparatus is capable of objectively measuring the eye refractive power of the examinee's eye in the eyeglasses worn state. about such an approach. According to JP 2001 161644 A, eight light sources are placed at a position where reflected light from the eyeglass lens by illuminating light is included in an anterior segment. Corneal reflection images 9' of eight light sources 9 are hereby reflected in the center of the screen and the positions of the reflection images are captured. Light sources 9 are placed at a position inclined less than 30° with respect to the measurement optical axis extending towards the eye E.

### CITATION LIST

### PATENT LITERATURE

### PATENT LITERATURE 1: JP-A-2001-161644

### SUMMARY OF THE INVENTION

An example is described in which if the eye refractive power in the eyeglasses worn state is measured in the above apparatus, only three of eight infrared light sources used in keratometry are used to illuminate the anterior segment in order to avoid reflected light from the eyeglass lens.

However, there are differences in the curve of an eyeglass lens and the fit of an eyeglass frame (for example, a lens placement angle with respect to an eyeglass frame) among individuals. Hence, in a case of the above configuration, an anterior segment image may include reflected light from the eyeglass lens. Moreover, it may become hard to perform alignment with the examinee's eye since the amount of light is not sufficient to illuminate the anterior segment as compared to a case where the eight infrared light sources are turned on. Moreover, it is troublesome to change settings between an eyeglasses not worn state and the eyeglasses worn state.

A technical issue of the present disclosure is to provide an eye refractive power measurement apparatus that can solve at least one of the problems of the known technology when the eye refractive power in the eyeglasses worn state is measured.

**In** order to solve the above issue, the present invention provides an eye refractive power measurement apparatus according to claim 1. Further developments are defined by the dependent claims.

The present invention provides:
An eye refractive power measurement apparatus includes: a measuring optical system for projecting measurement light onto the fundus of an examinee's eye, receiving reflected light therefrom, and accordingly objectively measuring the eye refractive power of the examinee's eye; an illuminating light source for illuminating the anterior segment of the examinee's eye in an eyeglasses worn state, the illuminating light source being placed at a position inclined 30° or greater with respect to a measurement optical axis of the measuring optical system, which extends towards the examinee's eye; an observing optical system configured to observe the anterior segment of the examinee's eye in the eyeglasses worn state, the anterior segment being illuminated by the illuminating light source; and a ring target projecting optical system configured to project a ring target in a central area of the cornea of the examinee's eye, wherein the illuminating light source is placed at a position further away from the measurement optical axis than the ring target projecting optical system. The eye refractive power measurement apparatus is capable of objectively measuring the eye refractive power of the examinee's eye in the eyeglasses worn state.

According to the present invention, it is possible to smoothly perform alignment with an examinee's eye even in the eyeglasses worn state.

### DESCRIPTION OF THE EMBODIMENTS

An embodiment according to the present disclosure is described hereinafter.

An eye refractive power measurement apparatus according to the embodiment may be an eye refractive power measurement apparatus that can objectively measure the eye refractive power of an examinee's eye in an eyeglasses worn state. The eye refractive power measurement apparatus may include, for example, an eye refractive power measuring optical system for objectively measuring the eye refractive power of the examinee's eye by receiving reflected light of measurement light projected onto the fundus of the examinee's eye. In this case, the eye refractive power measuring optical system may include, for example, a light projecting optical system that projects measurement light onto the fundus of the examinee's eye, and a light receiving optical system that receives fundus reflected light of the measurement light.

The eye refractive power measurement apparatus may include an observing optical system for observing a front image of the anterior segment of the examinee's eye. The observing optical system may be able to observe a front image of the anterior segment in the eyeglasses worn state. In this case, the observing optical system may include, for example, an imaging device for capturing an image of the anterior segment illuminated by anterior segment lighting.

The eye refractive power measurement apparatus may include an illuminating light source for illuminating the anterior segment of the examinee's eye in the eyeglasses worn state. The illuminating light source may be a dedicated anterior segment illuminating light source or an anterior segment illuminating light source that also serves another purpose.

The illuminating light source for illuminating the anterior segment in the eyeglasses worn state may, for example, be placed at a position inclined 30° or greater with respect to the measurement optical axis of the eye refractive power measuring optical system. Accordingly, artifacts occurring due to reflected light from the eyeglass lens are reduced irrespective of, for example, the curve of the eyeglass lens or the fit of the eyeglass frame. The placement angle of the illuminating light source may be determined as, for example, the angle between a straight line linking the center of the cornea of the examinee's eye placed at a proper working distance and the illuminating light source, and a measurement optical axis L1.

The eye refractive power measurement apparatus may include a ring target projecting optical system that projects a ring target in a central area of the cornea of the examinee's eye. The central area of the cornea of the examinee's eye may be a corneal area inward of an area that is 3.5 mm away from the center of the cornea of the examinee's eye. The ring target that is projected in the central area of the cornea of the examinee's eye may be, for example, a Mayer ring target. The ring target may be used to judge keratoconus and the like by an examiner's visual observation. Moreover, the ring target may be used to perform alignment with the examinee's eye. Moreover, the ring target may be used to measure the corneal shape. The projection of the ring target may be used to illuminate the anterior segment. The ring target projecting optical system may have a configuration that is used for at least any of the above purposes. Alternatively, the optical system may have a configuration that is used for two or more purposes. The ring target that is projected in the central area of the cornea is simply required to be a ring-shaped target. The ring target may be a continuous ring (for example, a circle) or a discontinuous ring (for example, a discontinuous ring made up of a plurality of points (for example, six points) placed on the circumference).

The illuminating light source for illuminating the anterior segment in the eyeglasses worn state may, for example, be placed at a position further away from the measurement optical axis than the ring projecting optical system. Accordingly, the anterior segment can be illuminated from outside the ring target.

The eye refractive power measurement apparatus may include a controller that controls the ring target projecting optical system. In this case, the controller may control the projection of the ring target by the ring target projecting optical system. For example, the controller may restrict the projection of the ring target when measuring the eye refractive power of the examinee's eye in the eyeglasses worn state. More specifically, the controller may restrict the projection of the ring target when set in a measurement mode for measuring the eye refractive power in the eyeglasses worn state. It is not necessarily required to set the measurement mode.

In this case, the controller may, for example, cause the illuminating light source for illuminating the anterior segment in the eyeglasses worn state to illuminate the anterior segment in a state where the projection of the ring target by the ring target projecting optical system is restricted. Furthermore, the controller may cause the measuring optical system to measure the eye refractive power of the examinee's eye during wear of eyeglasses, the examinee's eye being illuminated by the illuminating light source. More specifically, the controller may cause the illuminating light source for illuminating the anterior segment in the eyeglasses worn state to illuminate the anterior segment in the state where the projection of the ring target by the ring projecting optical system is restricted. Automatic alignment may be performed on the anterior segment where the projection of the ring target is restricted. After the automatic alignment is completed, the measuring optical system may measure the eye refractive power of the examinee's eye during wear of eyeglasses. Naturally, the embodiment is not limited to this and can also be applied to manual alignment.

The eye refractive power measurement apparatus may be provided with a detector that detects whether or not an eyeglass lens is worn for the examinee's eye. The detector may be, for example, an imaging device of the observing optical system. Alternatively, the detector may be a detector that is provided separately from the observing optical system. The detector may have a configuration that detects the worn/not worn state optically. Alternatively, the worn/not worn state may be detected by another method such as ultrasound or magnetism. The use of the imaging device of the observing optical system allows simplifying the configuration of the apparatus. Moreover, the detector may detect the worn/not worn state by, for example, detecting reflection from an eyeglass lens or eyeglass frame.

The controller may switch the measurement modes on the basis of a detection signal from the detector that detects the worn/not worn state. For example, the controller may switch between a first measurement mode for measuring the eye refractive power in the eyeglasses not worn state and a second measurement mode for measuring the eye refractive power in the eyeglasses worn state, on the basis of the detection signal. The second measurement mode may be set if the controller has determined that an eyeglass lens is worn, on the basis of the detection signal. Accordingly, the measurement modes can be switched smoothly.

The controller may control the projection of the ring target through the ring projecting optical system on the basis of the detection signal from the detector that detects the worn/not worn state. The controller may restrict the projection of the ring target if the controller has determined that an eyeglass lens is worn, on the basis of the detection signal.

The illuminating light source for illuminating the anterior segment in the eyeglasses worn state is not limited to the case of being placed at a position inclined 30° or greater with respect to the measurement optical axis of the eye refractive power measuring optical system. As long as a configuration is adopted in which the illuminating light source is placed at a position further away from the measurement optical axis than the ring projecting optical system, the embodiment can be applied to the configuration. For example, various control types illustrated above can also be used. The use of a point light source as the illuminating light source allows reducing artifacts caused by an eyeglass lens as compared to the projection of the ring target.

The eye refractive power measurement apparatus may include, for example, a mode switch that switches between the first measurement mode for measuring the eye refractive power in the eyeglasses not worn state and the second measurement mode for measuring the eye refractive power in the eyeglasses worn state.

The controller may associate a measurement result of the measuring optical system with the lens worn/not worn state and output the measurement result. For example, characters, colors, or symbols are conceivable as an output method. However, naturally, the output method is not limited to them. In this case, the controller may, for example, determine a measurement result of the measuring optical system according to the measurement mode and output the measurement result. Accordingly, the eyeglasses worn/not worn state can be easily determined in terms of the obtained measurement result.

### <Example>

An example according to the embodiment of the present disclosure is described hereinafter with reference to the drawings. In the following example, an eye refractive power measurement apparatus 1 that objectively measures the eye refractive power of an examinee's eye E is illustrated by example. Examples of the eye refractive power measurement apparatus include an autorefractometer and an eye aberrometer.

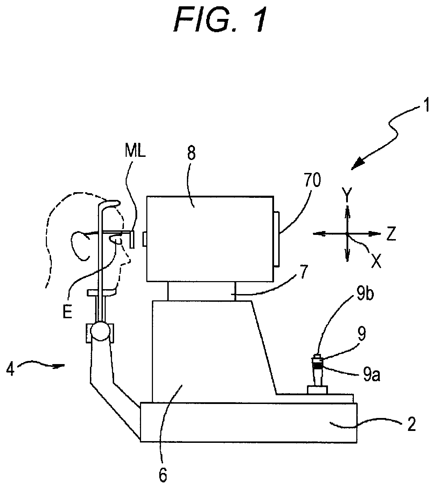

Firstly, an example of an external configuration of the eye refractive power measurement apparatus 1 is illustrated with reference to Fig. 1. The eye refractive power measurement apparatus 1 illustrated in Fig. 1 is what is called a tabletop. The eye refractive power measurement apparatus 1 mainly includes a measuring unit 8. The measuring unit 8 is provided with at least an optical system that is used when eye characteristics are measured. The details are described below. The eye refractive power measurement apparatus 1 may be a portable.

Moreover, in the example of Fig. 1, the eye refractive power measurement apparatus 1 further includes a base 2, a face support unit 4, a movable carriage 6, a driving unit 7, a joystick 9, and a monitor 70.

The movable carriage 6 is supported by the base 2. The movable carriage 6 moves in an up-down direction (Y direction) and a front-rear direction (Z direction) on the base 2 with the operation of the joystick 9. Moreover, the face support unit 4 is fixed to the base 2. As illustrated in Fig. 1, the face support unit 4 is used to support the face of the examinee with the examinee's eye E facing the measuring unit 8. The driving unit 7 moves the measuring unit 8 in a right-left direction (X direction), the up-down direction (Y direction), and the front-rear direction (Z direction) with respect to the examinee's eye E. An examiner rotates a rotating knob 9a provided to the joystick 9; accordingly, the driving unit 7 moves the measuring unit 8 in the Y direction. Moreover, a switch 9b is provided on the top of the joystick 9. Various pieces of information such as an observation image of the examinee's eye E taken by the measuring unit 8, and a measurement result of the examinee's eye E by the measuring unit 8 are displayed on the monitor 70.

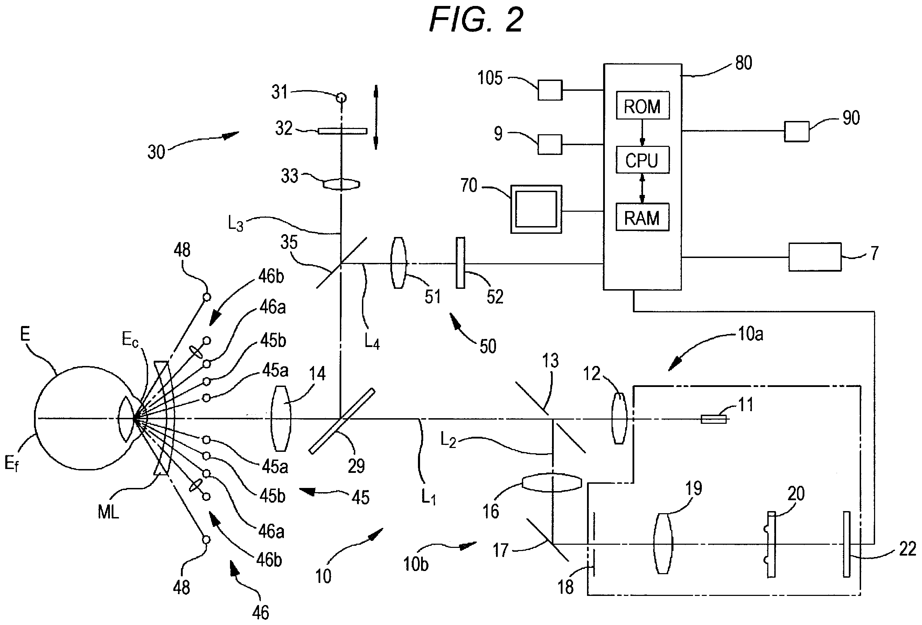

Next, optical systems provided to the measuring unit 8 of the eye refractive power measurement apparatus 1 are described with reference to Fig. 2. The measuring unit 8 mainly includes a measuring optical system 10 and an observing optical system (imaging optical system) 50. Moreover, the measuring unit 8 in Fig. 2 includes a fixation target presenting optical system 30, a ring target projecting optical system 45, a working distance target projecting optical system 46, and an illuminating light source 48.

The measuring optical system 10 illustrated in Fig. 2 is used to measure the eye refractive power of the examinee's eye. In the measuring optical system 10 illustrated in Fig. 2, a measurement axis is an optical axis indicated by L1. The measuring optical system 10 illustrated in Fig. 2 includes a light projecting optical system 10a and a light receiving optical system 10b. The light projecting optical system 10a projects measurement light flux onto a fundus Er of the examinee's eye E through the pupil of the examinee's eye E. Moreover, the light receiving optical system 10b captures fundus reflected light by the measurement light flux as a ring-shaped fundus reflection image with a two-dimensional imaging device 22 (an example of a detector). The ring image may be a continuous ring image. Alternatively, the ring image may be a discontinuous ring image (for example, a discontinuous ring image made up of a plurality of points).

For example, the light projecting optical system 10a includes a measurement light source 11, a relay lens 12, a hole mirror 13, and an objective lens 14. The light receiving optical system 10b shares the hole mirror 13 and the objective lens 14 with the light projecting optical system 10a. Moreover, the light receiving optical system 10b includes a relay lens 16, a total reflection mirror 17, a light receiving aperture 18, a collimator lens 19, a ring lens 20, and the two-dimensional imaging device 22 (hereinafter referred to as the imaging device 22). These measuring optical systems 10 are provided in a transmission direction of a dichroic mirror 29 in the example of Fig. 2.

The ring lens 20 of the light receiving optical system 10b is an optical element for shaping fundus reflected light into a ring form. The ring lens 20 includes a lens part formed into a ring shape, and a light shielding part having a coating for light shielding applied to an area other than the lens part. Moreover, the ring lens 20 has a positional relationship that is optically conjugate to the pupil of the examinee's eye E. The ring-shaped fundus reflected light (that is, a two-dimensional pattern image) through the ring lens 20 is received by the imaging device 22. The imaging device 22 outputs image information on the received two-dimensional pattern image to a controller 80. Accordingly, the two-dimensional pattern image can be displayed on the monitor 70. Moreover, the refractive power of the examinee's eye E can be calculated on the basis of the two-dimensional pattern image. A photo detector such as an area CCD can be used as the imaging device 22.

The measuring optical system 10 is not limited to the above optical system. Various configurations that are known as configurations for objectively measuring the eye refractive power may be used. Moreover, the measuring optical system 10 that objectively measures the eye refractive power may be an eye aberration measuring optical system that can also measure high-order aberration of the examinee's eye. The eye aberration measuring optical system may have, for example, a configuration including a Shack-Hartmann sensor. Naturally, an apparatus of another measurement technique (for example, an apparatus of a phase contrast technique that projects a slit) may be used.

The beam splitter 29 is placed between the objective lens 14 and the hole mirror 13. The beam splitter 29 guides light flux from the fixation target presenting optical system 30 described below to the examinee's eye E, and guides reflected light from an anterior segment Ec of the examinee's eye E to the observing optical system 50. Moreover, the beam splitter 29 reflects part of fundus reflected light emitted from the light source 11 and reflected by the fundus Er and guides it to the observing optical system 50. At the same time, the beam splitter 29 transmits the other part of the fundus reflected light and guides it to the light receiving optical system 10b.

The fixation target presenting optical system 30 is a fixation optical system for causing the examinee's eye E to fixate. The fixation target presenting optical system 30 includes a visible light source 31, a fixation target plate 32, a light projecting lens 33, the beam splitter 29, and the objective lens 14. The visible light source 31 is turned on to present a fixation target of the fixation target plate 32 to the examinee's eye E. The visible light source 31 and the fixation target plate 32 are configured in such a manner as to be movable in an optical axis L3 direction by an unillustrated sliding mechanism. The visible light source 31 and the fixation target plate 32 are moved in the optical axis L3 direction to fog the examinee's eye E.

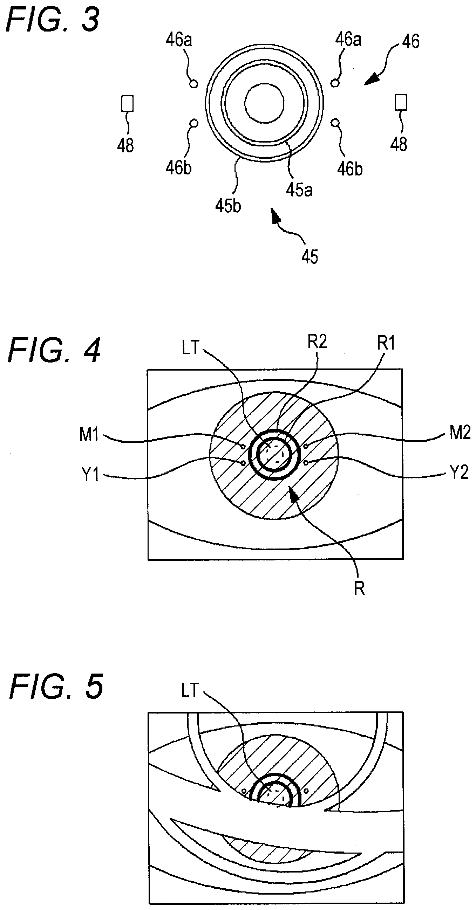

Fig. 3 is a schematic diagram illustrating an example of an examinee-side housing surface of the measuring unit 8. The ring target projecting optical system 45 and the working distance target projecting optical system 46, which are examples of alignment target projecting optical systems, are placed in front of the anterior segment of the examinee's eye E. The ring target projecting optical system 45 is used to project a ring target in a central area of the cornea of the examinee's eye. The ring target projecting optical system 45 may be, for example, a projecting optical system that projects a Mayer ring onto the examinee's eye.

In terms of the placement angle of the ring target projecting optical system 45, a projecting optical system 45a for projecting an inner ring target may, for example, be configured in such a manner as to project a ring target R1 in an area that is a first distance away from the center of the cornea of the examinee's eye. In this case, the ring target projecting optical system 45a may, for example, be configured in such a manner as to be placed, inclined a first angle (for example, 16 degrees) with respect to the measurement optical axis L1. The first distance can be set, for example, between 1.5 mm and 2.7 mm.

A projecting optical system 45b for projecting an outer ring target may, for example, be configured in such a manner as to project a ring target R2 in an area that is a second distance away from the center of the cornea of the examinee's eye. The second distance is different from the first distance. In this case, the ring target projecting optical system 45a may, for example, be configured in such a manner as to be placed, inclined a second angle (for example, 27 degrees) with respect to the measurement optical axis L1, the second angle being different from the first angle. The second distance can be set, for example, between 2.8 mm and 3.5 mm.

In the example, the optical system (45a and 45b) that projects two concentric ring targets is described. However, the embodiment is not limited to this example. The ring target projecting optical system 45 may be configured in such a manner as to project a single ring target. Alternatively, the optical system may be configured in such a manner as to project three or more concentric ring targets. Moreover, the area on the cornea where the ring target is projected is not limited to the above area. The angles of inclination change depending on a preset proper working distance between the examinee's eye and the measuring unit 8. Hence, the angles of inclination are not limited to the above angles.

In the example, the ring target projecting optical system 45 projects infrared light (for example, near-infrared light) in a ring form onto the cornea Ec. As a result, a ring target image is formed on the cornea Ec as illustrated in Fig. 4. The corneal apex (substantial corneal apex) is detected as the center position of the ring image.

The ring target that is projected onto the cornea may, for example, be used to judge keratoconus and the like by the examiner's visual observation. Moreover, the ring target may be used as an alignment target for performing alignment with the examinee's eye. Moreover, the ring target may be used as a target for measuring the corneal shape. The ring projecting optical system 45 may be used as anterior segment lighting that illuminates the anterior segment of the examinee's eye E.

The working distance target projecting optical system 46 is, for example, used to project a target for performing alignment in a working distance direction. An alignment target projected on the cornea is used for alignment (for example, automatic alignment, alignment detection, or manual alignment) with the examinee's eye. A projecting optical system 46a is an optical system for projecting finite-distance targets Y1 and Y2 onto the cornea Ec of the examinee's eye E. A projecting optical system 46b is an optical system for projecting infinity targets M1 and M2 onto the cornea Ec of the examinee's eye E. The working distance target projecting optical system 46 uses, for example, infrared light (for example, near-infrared light). In the example, the working distance target projecting optical system 46 is formed at a position outside the ring targets. However, the embodiment is not limited to this example. For example, a projecting optical system that projects an infinity target, which is provided to a break in the ring target projecting optical system 45, allows alignment in the working distance direction, using the ring target and the infinity target.

### <Anterior Segment Illuminating Light Source for Eyeglasses Worn state>

The illuminating light source 48 may be used as, for example, a light source for illuminating the anterior segment of the examinee's eye in the eyeglasses worn state. The illuminating light source 48 is placed at a position that is set in such a manner as to allow avoiding inclusion of reflected light from the eyeglass lens by illuminating light in an anterior segment image. Accordingly, alignment can be smoothly performed on the examinee's eye even in the eyeglasses worn state. The illuminating light source 48 may be an infrared light source or a visible light source.

In this case, the illuminating light source 48 may, for example, be placed at a position inclined 30° or greater with respect to the measurement optical axis L1 of the measuring optical system 10. Accordingly, a reflection image from the eyeglass lens by the illuminating light source 48 is formed at a position away from the measurement optical axis L1. Hence, the reflection image is not included in the anterior segment image. Alternatively, even if the reflection image is included in the anterior segment image, the reflection image is formed in a peripheral portion in the anterior segment image. As a result, artifacts that occur when alignment is performed on the examinee's eye are reduced. If an artifact is formed in the peripheral portion in the anterior segment image, a central portion in the anterior segment image does not include an artifact. Hence, alignment with, for example, the center of the cornea or the center of the pupil is easy.

An angle of incidence to the eyeglass lens is increased. Hence, alignment using the observing optical system 50 is hardly influenced by reflected light from the eyeglass lens irrespective of the curve of the eyeglass lens or the fit of the eyeglass frame. As an aspect of the artifact reduction, more preferably, the illuminating light source 48 may be placed at a position inclined 35° or greater with respect to the measurement optical axis L1 of the measuring optical system 10. Accordingly, the possibility that an anterior segment image does not include a reflection image from the lens increases.

In the example, the illuminating light source 48 may, for example, be placed at a position further away from the measurement optical axis L1 than the ring target projecting optical system 45. The projecting optical system 45 is placed at a position close to the measurement optical axis L1. Hence, reflected light from the eyeglass lens by the ring target projecting optical system 45 is likely to move toward a test window. As a result, the reflected light from the eyeglass lens is likely to be included in an imaging device 52. In contrast, the illuminating light source 48 is further away from the measurement optical axis L1 than the ring target projecting optical system 45. Hence, reflected light from the eyeglass lens by the illuminating light source 48 is unlikely to move toward the test window. As a result, the reflected light from the eyeglass lens is unlikely to be included in the anterior segment. The illuminating light source 48 may, for example, be configured in such a manner as to be placed, inclined a greater angle with respect to the measurement optical axis L1 than the ring target projecting optical system 45 (set at 50 degrees to 60 degrees in Fig. 2). In this case, the illustrated placement angle is simply an example, and the angle can be appropriately set, considering the set proper working distance and the influence of the reflected light from the eyeglass lens by the illuminating light source 48.

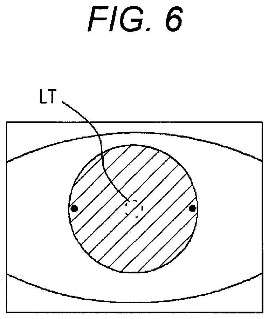

In a case of a Mayer ring projecting optical system that is generally used for an eye refractive power measurement apparatus in an experiment by the inventors, a reflection image from an eyeglass lens by the projecting optical system resulted in appearing in an anterior segment image (refer to, for example, Fig. 5). On the other hand, it was found that the appearance of a reflection image from the eyeglass lens could be reduced by turning off ring lighting of the Mayer ring projecting optical system and placing, as anterior segment lighting, an illuminating light source outside the Mayer ring projecting optical system (refer to, for example, Fig. 6).

In the above configuration, if the examinee's eye in the eyeglasses worn state is measured, smooth alignment with the examinee's eye in the eyeglasses worn state can be achieved by turning off the ring target projecting optical system 45 and using the illuminating light source 48 as the anterior segment lighting. Moreover, if the examinee's eye in the eyeglasses not worn state is measured, the use of the ring target projecting optical system 45 allows alignment using a ring target and a check for keratoconus and the like (the illuminating light source 48 may be on).

The illuminating light source 48 may be, for example, a point light source. Furthermore, in the illuminating light source 48, point light sources may be symmetrically placed right and left about the measurement optical axis L1. The point light source can reduce the area of a reflection image. Hence, artifacts occurring due to reflection from an eyeglass lens can be reduced more reliably. Naturally, the illuminating light source 48 is not limited to a point light source. The illuminating light source 48 may be a ring-shaped light source or line-shaped light source.

The illuminating light source 48 may also serve as anterior segment lighting that illuminates the anterior segment when the corneal diameter or pupil diameter of the examinee's eye is measured. Generally, the anterior segment lighting that is used when the corneal diameter or pupil diameter is measured is placed at a position relatively away from the measurement optical axis L1 to prevent a target from being formed on the boundary between the cornea and the sclera or on the boundary between the pupil and the iris. Hence, the anterior segment lighting that is used when the corneal diameter or pupil diameter is measured is used as the anterior segment lighting when the eye refractive power of the examinee's eye in the eyeglasses worn state is measured. Alignment is then performed on the anterior segment of the examinee's eye. Accordingly, reflected light from the eyeglass lens is reduced. As a result, smooth alignment can be performed.

The observing optical system (imaging optical system) 50 includes the imaging device 52 that captures a front image of the anterior segment of the examinee's eye E. In the embodiment, the observing optical system 50 shares the objective lens 14 and the beam splitter 29 with the fixation target presenting optical system 30. Moreover, the observing optical system 50 includes a half mirror 35, an imaging lens 51, and the two-dimensional imaging device 52 (hereinafter referred to as the imaging device 52). The imaging device 52 is a photo detector having an imaging surface placed at a position substantially conjugate to the anterior segment of the examinee's eye E. The imaging device 52 captures a front image of the anterior segment of the examinee's eye E. The output from the imaging device 52 is inputted into the controller 80. As a result, a live image of the front image captured by the imaging device 52 is displayed as an observation image on the monitor 70. In the embodiment, the observing optical system 50 also serves as an optical system that detects alignment target images (the ring targets and the infinity targets in the embodiment). The alignment target images are formed by the target projecting optical systems 45 and 46 on the cornea Ec of the examinee's eye E. The positions of the alignment target images are detected on the basis of imaging results of the alignment target images by the imaging device 52. The observing optical system 50 can capture a front image of the anterior segment of the examinee's eye in the eyeglasses worn state illuminated by the illuminating light source 48.

Next, a control system of the eye refractive power measurement apparatus 1 is described with reference to Fig. 2. In the eye refractive power measurement apparatus 1, the controller 80 controls each unit. The controller 80 is a processing device (processor) having electronic circuits that perform control processes of each unit and computing processes. The controller 80 is realized by, for example, a CPU (Central Processing Unit) and memory. The controller 80 is electrically connected to each of the light sources 11 and 31, the imaging devices 22 and 52, the movable carriage 6, the driving unit 7, the joystick 9, the monitor 70, an operating unit 90, and a memory 105.

The memory 105 may be a rewritable nonvolatile storage device. At least a program for causing the controller 80 to execute a measurement operation may be stored in the memory 105.

The controller 80 controls each member of the eye refractive power measurement apparatus 1 on the basis of an operation signal outputted from the operating unit 90. The operating unit 90 may be a pointing device such as a touchscreen or a mouse. Alternatively, the operating unit 90 may be, for example, a keyboard.

In the example, the controller 80 may be able to automatically or manually switch between a first measurement mode for measuring the eye refractive power in the eyeglasses not worn state and a second measurement mode for measuring the eye refractive power in the eyeglasses worn state. For example, the controller 80 may switch the measurement modes on the basis of an operation signal from the operating unit 90.

Furthermore, the controller 80 may control the ring target projecting optical system 45 in accordance with the switched mode. For example, the controller 80 may project the ring target onto the examinee's eye if the first measurement mode is set. For example, the controller 80 may restrict the projection of the ring target onto the examinee's eye if the second measurement mode is set.

If the projection of the ring target onto the examinee's eye is controlled, for example, the turning-on/off of a light source of the ring target projecting optical system 45 may be controlled. Alternatively, shielding or non-shielding by, for example, a shutter placed between the light source and the examinee's eye may be controlled. Moreover, configurations of the case where the projection of the ring target is restricted include a configuration where projected light of the ring target is dimmed to a degree that reflected light from an eyeglass lens by the ring target does not influence alignment, in addition to a configuration where projection onto the examinee's eye is completely restricted. In this case, the projection of all the ring targets may be restricted. Alternatively, the projection of part of the ring targets may be restricted.

In the example, the controller 80 may be able to automatically or manually switch between a first alignment mode for aligning the measuring unit 8 with the anterior segment of the examinee's eye in the eyeglasses not worn state and a second alignment mode for aligning the measuring unit 8 with the anterior segment of the examinee's eye in the eyeglasses worn state. For example, the controller 80 may switch the alignment modes on the basis of an operation signal from the operating unit 90.

Furthermore, the controller 80 may switch alignment detection methods for detecting the alignment state of the measuring unit 8 with the examinee's eye in accordance with the switched mode. For example, the controller 80 may detect the alignment state, using the ring target projected on the anterior segment of the examinee's eye if the first alignment mode is set. For example, the controller 80 may detect the alignment state, using a feature portion (for example, the pupil or iris) of the anterior segment of the examinee's eye illuminated by the illuminating light source 48 if the second alignment mode is set. Moreover, the controller 80 may detect the alignment state, using a bright spot on the cornea formed by the illuminating light source 48 on the anterior segment if the second alignment mode is set.

The controller 80 may perform automatic alignment of the measuring unit 8 with the examinee's eye by controlling the driving unit 7 on the basis of the detection result of the alignment state detected as described above. Moreover, the controller 80 may notify the examiner of the detection result of the alignment state detected as described above (for example, the detection result may be electronically displayed on the monitor 70).

In terms of the mode settings, the first alignment mode may be set in conjunction with switching to the first measurement mode. Alternatively, the second alignment mode may be set in conjunction with switching to the second measurement mode.

Moreover, the embodiment is not limited to the above. A first mode including a combination of the first measurement mode and the first alignment mode may be able to be set. Alternatively, a second mode including a combination of the second measurement mode and the second alignment mode may be able to be set. Moreover, each of the first measurement mode, the first alignment mode, the second measurement mode, and the second alignment mode may be able to be set independently.

### <Operation of Apparatus>

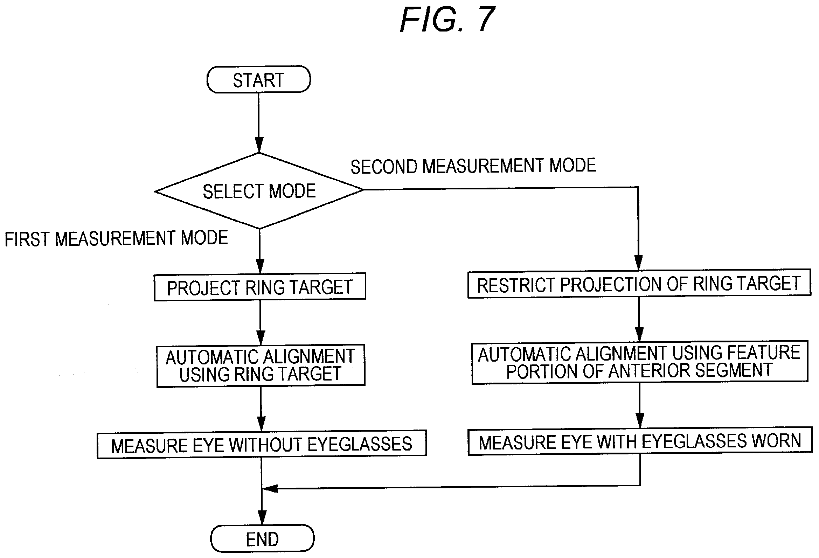

An example of the operation of the apparatus including the above configuration is described. Fig. 7 is a flowchart explaining the operation of the apparatus according to the example. In the following description, a case of measuring the eye refractive power of an examinee's eye in the eyeglasses not worn state that is an examinee's eye for which an eyeglass lens is not worn, and a case of measuring the eye refractive power of an examinee's eye in the eyeglasses worn state that is an examinee's eye for which an eyeglass lens is worn are described. Here, an examinee's eye with an eyeglass lens placed in front of the eye via an eyeglass frame is assumed to be the examinee's eye in the eyeglasses worn state.

### <First Measurement Mode>

If the examinee's eye in the eyeglasses not worn state is measured, the examiner operates the operating unit 90 to set the first measurement mode. In the first measurement mode, for example, the ring targets by the ring target projecting optical system 45 and the targets by the working distance target projecting optical system 46 are projected onto the examinee's eye. The ring targets are used to illuminate the anterior segment and detect alignment.

The examiner fixes the face of the examinee to the face support unit 4. In addition, the examiner instructs the examinee to fixate the fixation target of the fixation target plate 32. Next, the alignment of the measuring unit 8 with the examinee's eye is performed in the X, Y, and Z directions. The examiner operates the joystick 9 and the rotating knob 9a while observing the monitor 70. In this manner, rough alignment is performed. In terms of the rough alignment, automatic alignment may be performed, using, for example, a camera that can photograph a wide area of the face of the examinee.

The controller 80 causes the monitor 70 to display an observation image of the anterior segment captured through the observing optical system 50 whenever necessary (refer to Fig. 4). In other words, a front image (live image) of the anterior segment taken in substantially real time is displayed on the monitor 70. A reticle mark LT indicates the position of a measurement axis in the measuring unit 8 (the measurement optical axis L1 of the measuring optical system 10 in the embodiment).

The rough alignment is performed to enter a state where the imaging device 52 captures the ring target images R1 and R2 by the ring target projecting optical system 45 and the infinity targets M1 and M2 and the infinite-distance targets Y1 and Y2 by the working distance target projecting optical system 46. The controller 90 then controls the drive of the driving unit 7 on the basis of an imaging signal from the imaging device 52. Accordingly, the measuring unit 8 moves in the X-Y direction or the Z direction. In this manner, detailed alignment of the measuring unit 8 with the examinee's eye is performed.

The controller 80 calculates coordinates of the center positions of the ring target images R1 and R2 detected by the imaging device 52. Accordingly, the state of alignment with the examinee's eye in the up-down and right-left directions is obtained. In this case, the coordinates of the center position of at least one of the ring target images R1 and R2 may be used. If both of the ring target images R1 and R2 are used, an average position may be used. The position of the center of the cornea can be obtained by detecting the center position of the ring target image.

If the measuring unit 8 deviates in the Z (working distance) direction from the examinee's eye, the image spacing between the infinity targets M1 and M2 hardly changes. In contrast, the image spacing between the finite-distance targets Y1 and Y2 changes. Using this characteristics, the controller 80 obtains the alignment state with the examinee's eye in the working distance direction (refer to JP-A-6-46999 for details). A configuration and detection method for detecting alignment in the Z direction is not limited to the above. For example, the level of blurring of the ring target image may be used.

### <Eye Refractive Power Measurement>

After alignment is completed, then measurements are made by refractometry. In this case, measurement light from the measurement light source 11 is projected onto the fundus through the measuring optical system 10. Fundus reflected light by the measurement light is received by the imaging device 22 through the measuring optical system 10. In this case, preliminary measurements of the eye refractive power are made. The light source 31 and the fixation target plate 32 then move in an optical axis L2 direction on the basis of a result of the preliminary measurements. Accordingly, the examinee's eye is fogged. The eye refractive power of the fogged examinee's eye is then measured.

In the measurements (and the preliminary measurements) of the eye refractive power, the controller 80 processes an output signal from the imaging device 22. Accordingly, the eye refractive power is obtained. The output signal from the imaging device 22 is stored as image data (a measurement image) in the memory 105. The controller 80 then locates the position of the ring image for each meridian of the ring on the basis of the image data stored in the memory 105. Next, the controller 80 approximates an ellipse, using, for example, the least-squares method, on the basis of the located image position of the ring image. The controller 80 then obtains refractive error in each meridian direction from the shape of the approximated ellipse. The eye refraction values (S (spherical power), C (cylindrical power), and A (astigmatic axial angle)) of the examinee's eye E are computed on the basis of the refractive error. The measurement results are then displayed on the monitor 70. Moreover, the controller 80 may store the measurement results of, for example, the eye refraction values in the memory 105. In this case, the controller 80 may display a determination display indicating that the examinee's eye in the eyeglasses not worn state has been measured, together with the measurement results.

The controller 80 may further measure the corneal shape of the examinee's eye on the basis of the ring target and display the measurement result of the corneal shape on the monitor 70 when measuring the eye refractive power of the examinee's eye in the eyeglasses not worn state.

### <Second Measurement Mode>

If measuring the examinee's eye in the eyeglasses worn state, the examiner operates the operating unit 90 to set the second measurement mode. In the second measurement mode, for example, the projection of the ring targets by the ring target projecting optical system 45 and the targets by the working distance target projecting optical system 46 is restricted. Illuminating light from the illuminating light source 48 is then projected onto the examinee's eye. In this case, only the projection of the ring target may be restricted. The illuminating light from the illuminating light source 48 is, for example, used to observe an anterior segment image. Alignment with the examinee's eye using the anterior segment image is then performed. In the following description, specific descriptions of the same parts as the first measurement mode are omitted.

In this case, the projection of the ring target and the infinity target is restricted. Accordingly, the influence of reflected light from an eyeglass lens ML on the anterior segment observation image due to these targets can be reduced. At the same time, the anterior segment is illuminated by the illuminating light source 48. Accordingly, as a result, the anterior segment observation image can be excellently observed (refer to, for example, Fig. 6).

Rough alignment is performed to enter a state where an image of a pupil portion P of the examinee's eye is captured by the imaging device 52. The controller 90 then controls the drive of the driving unit 7 on the basis of an imaging signal from the imaging device 52. Accordingly, the measuring unit 8 moves in the X-Y direction or the Z direction. In this manner, detailed alignment of the measuring unit 8 with the examinee's eye is performed.

The controller 80 analyzes the pupil portion P of which image has been captured by the imaging device 52, through image processing. The pupil center coordinates in the pupil portion P are then calculated. Accordingly, the alignment state with the examinee's eye in the up-down and right-left directions is obtained. It is not necessarily required to calculate the pupil center coordinates. Automatic alignment may be performed in such a manner that measurement light flux from the measuring optical system 10 passes the pupil region.

If the measuring unit 8 deviates in the Z (working distance) direction from the examinee's eye, the anterior segment image (for example, the pupil portion P) becomes blurry. Using this characteristic, the controller 80 may obtain the alignment state of the measuring unit 8 with the examinee's eye in the working distance direction. For example, the controller 80 may move the measuring unit 8 in the Z direction, and acquire an evaluation value of the edge (the level of blurring) of the anterior segment image at each Z position and then move the measuring unit 8 taking a position having a high evaluation value as an appropriate position.

### <Eye Refractive Power Measurement>

After alignment is completed, then measurements are made by refractometry. In this case, measurement light from the measurement light source 11 is projected onto the fundus through the measuring optical system 10 and the eyeglass lens ML. Fundus reflected light by the measurement light is received by the imaging device 22 through the eyeglass lens ML and the measuring optical system 10. In this case, preliminary measurements of the eye refractive power are made. The eye refractive power of the fogged examinee's eye is then measured.

The controller 80 computes eye refraction values of the examinee's eye on the basis of the imaging result of the imaging device 22, and displays the measurement results on the monitor 70. Moreover, the controller 80 may store the measurement results of the eye refraction values in the memory 105. In this case, the controller 80 may display a determination display indicating that the examinee's eye in the eyeglasses worn state has been measured, together with the measurement results.

The eye refractive power in the eyeglasses worn state is measured as described above. Accordingly, the eye refractive power in a corrected state by an eyeglass lens can be measured. Accordingly, it is possible to, for example, evaluate whether or not the examinee's eye is appropriately corrected by the eyeglass lens.

If the eye refractive power of the examinee's eye in the eyeglasses worn state is measured, the diopter of the examinee's eye is corrected to some extent. Light flux emitted from the examinee's eye is close to parallel light flux. Hence, it is also conceivable that the emitted light flux has a tendency to be hard to be influenced by misalignment in the Z direction. Hence, in terms of the Z direction, an allowable alignment range, which is wider than in a case of roughly adjusting alignment to some extent, or in a case of performing automatic alignment on the examinee's eye in the eyeglasses not worn state, may be set.

In the above description, the illuminating light source 48 may also be projected onto the examinee's eye in the first measurement mode. In this case, the controller 80 may, for example, increase the amount of illumination light of the illuminating light source 48 in the second measurement mode as compared to the amount of illumination light in the first measurement mode. Accordingly, it is possible to, for example, compensate for a reduction in the amount of light due to the restriction of the ring target.

In the above description, the measurement modes are switched on the basis of an operation signal from the operating unit 90. However, the switching of the measurement modes is not limited to this. The measurement modes may be switched automatically. For example, the controller 80 may switch the measurement modes on the basis of an imaging signal outputted from the observing optical system 50. Here, the controller 80 may determine whether or not an eyeglass lens is worn, on the basis of an imaging signal outputted form the observing optical system 50 and switch the measurement modes on the basis of the determination result.

In this case, for example, the ring target from the ring target projecting optical system 45 is projected onto the examinee's eye in the eyeglasses worn state. At this point in time, the fact that the luminance value of the entire anterior segment image increases as compared to in the eyeglasses not worn state since much of reflected light from the eyeglass lens enters the imaging device 52 of the observing optical system 50 may be used. Here, if the area of pixels that exceed a predetermined threshold in the anterior segment image exceeds a tolerance, the controller 80 may determine that an eyeglass lens is worn and set the second measurement mode. Moreover, if the area of pixels that exceed the predetermined threshold in the anterior segment image falls below the tolerance, the controller 80 may determine that an eyeglass lens is not worn and set the first measurement mode. Naturally, the method for determining whether or not an eyeglass lens is worn is not limited to this. For example, upon alignment the controller 80 may determine the presence or absence of an eyeglass frame of which image is captured by the imaging device 52 through image processing and switch the measurement modes on the basis of the determination result. Moreover, the controller 80 may determine the presence or absence of lens reflection in a center area of a captured image captured by the imaging device 52.

In the above description, the optical system that projects the ring target is illustrated by example as the target projecting optical system placed at a position closer to the measurement optical axis L1 than the illuminating light source 48. However, the target projecting optical system is not limited to the optical system that projects the ring target. The embodiment can also be applied to an optical system that projects another target (for example, a point target or line target).

In the above description, the configuration where the ring target projecting optical system is placed at a position closer to the measurement optical axis L1 than the illuminating light source 48 is illustrated. However, the embodiment can also be applied to a configuration where a ring target projecting optical system is additionally placed at a position farther from the measurement optical axis L1 than the illuminating light source 48. In this case, the illuminating light source 48 may also serve as the ring target projecting optical system in this configuration.

In the above description, the illuminating light source 48 may be a plurality of illuminating light sources placed, inclined at individually different angles with respect to the measurement optical axis L1. In this case, the plurality of illuminating light sources may be selectively turned on. For example, if a reflection bright spot from an eyeglass lens appears upon observation of an anterior segment image, the examiner may operate the operating unit 90, turn off the illuminating light source at an angle corresponding to the reflection bright spot, and turn on the illuminating light source at another angle.

### <Application to Other Apparatuses>

Moreover, in the above description, the eye refractive power measurement apparatus that objectively measures the eye refractive power of an examinee's eye is illustrated by example as the eye refractive power measurement apparatus. However, the eye refractive power measurement apparatus is not limited to this illustration. The embodiment can also be applied to an eye refractive power measurement apparatus that subjectively measures the eye refractive power of an examinee's eye. In this case, for example, automatic alignment of a subjective test machine and detection of ocular alignment based on an anterior segment image that is acquired upon subjective measurement of the eye refractive power are conceivable.

Moreover, in the above description, the eye refractive power measurement apparatus that measures the eye refractive power during wear of eyeglasses is illustrated by example as the ophthalmic apparatus. However, the ophthalmic apparatus is not limited to this illustration. The embodiment can also be applied to an ophthalmic examination apparatus that examines an examinee' eye during wear of eyeglasses. The ophthalmic examination apparatus may include, for example, an examination optical system for examining an examinee's eye. The examination optical system applies (projects) light to the examinee's eye E through an eyeglass lens and receives reflected light from the examinee's eye E by a detector (for example, a photo detector or an imaging device) through the eyeglass lens. In this case, the ophthalmic examination apparatus may be, for example, a non-contact ocular axial length measurement apparatus or a fundus photographing apparatus.

### BRIEF DESCRIPTION OF THE DRAWINGS

Fig. 1 is a diagram illustrating an example of an external configuration of an eye refractive power measurement apparatus according to the example.
Fig. 2 is a diagram illustrating an example of optical systems of the eye refractive power measurement apparatus according to the example.
Fig. 3 is a schematic diagram illustrating an example of an examinee-side housing surface of a measuring unit according to the example.
Fig. 4 is a diagram illustrating an example of an anterior segment observation image according to the example.
Fig. 5 is a diagram illustrating an example of an anterior segment observation screen according to the example.
Fig. 6 is a diagram illustrating an example of the anterior segment observation screen according to the example.
Fig. 7 is a flowchart illustrating an example of the operation of the eye refractive power measurement apparatus according to the example.

### LIST OF REFERENCE NUMERALS

- 1: Eye refractive power measurement apparatus
- 45: Ring target projecting optical system
- 48: Illuminating light source
- 50: Observing optical system
- 80: Controller

## Claims

1. An eye refractive power measurement apparatus (1) comprising:
a measuring optical system (10) for projecting measurement light onto the fundus of an examinee's eye (E), receiving reflected light therefrom, and accordingly objectively measuring the eye refractive power of the examinee's eye (E);
an illuminating light source (48) for illuminating the anterior segment of the examinee's eye (E) in an eyeglasses worn state, the illuminating light source (48) being placed at a position inclined 30° or greater with respect to a measurement optical axis (L1) of the measuring optical system (10), which extends towards the examinee's eye (E);
an observing optical system (50) configured to observe the anterior segment of the examinee's eye (E) in the eyeglasses worn state, the anterior segment being illuminated by the illuminating light source (48); and
a ring target projecting optical system (45) configured to project a ring target (R1, R2) in a central area of the cornea of the examinee's eye (E), wherein the illuminating light source (48) is placed at a position further away from the measurement optical axis (L1) than the ring target projecting optical system (45), wherein
the eye refractive power measurement apparatus (1) is capable of objectively measuring the eye refractive power of the examinee's eye (E) in the eyeglasses worn state.

2. The eye refractive power measurement apparatus (1) according to claim 1, comprising:
mode switching means configured to switch between a first measurement mode for measuring the eye refractive power in an eyeglasses not worn state and a second measurement mode for measuring the eye refractive power in the eyeglasses worn state; and
control means (80) configured to control the ring target projecting optical system (45) in accordance with the measurement mode switched by the mode switching means.

3. The eye refractive power measurement apparatus (1) according to claim 2, comprising a detector configured to detect whether or not an eyeglass lens is worn for the examinee's eye (E), wherein the eye refractive power measurement apparatus (1) is configured to switch the measurement modes on the basis of a detection signal from the detector.

4. The eye refractive power measurement apparatus (1) according to any of claims 1 to 3, comprising alignment mode switching means configured to switch between a first alignment mode for aligning a measuring unit including the measuring optical system (10) with the anterior segment of the examinee's eye (E) in the eyeglasses not worn state and a second alignment mode for aligning the measuring unit with the anterior segment of the examinee's eye (E) in the eyeglasses worn state, wherein
the eye refractive power measurement apparatus (1) is configured to
detect an alignment state using the ring target (R1, R2) projected by the ring target projecting optical system (45) upon the first alignment mode being set, and
detect an alignment state using a feature portion of the anterior segment of the examinee's eye (E) illuminated by the illuminating light source (48) upon the second alignment mode being set.

5. The eye refractive power measurement apparatus (1) according to any of claims 1 to 4, wherein the illuminating light source (48) also serves as anterior segment lighting upon measurement of the corneal diameter or pupil diameter of the examinee's eye (E).

6. The eye refractive power measurement apparatus (1) according to any of claims 1 to 5, wherein the illuminating light source (48) is a point light source.

7. The eye refractive power measurement apparatus (1) according to claim 1, comprising control means configured to illuminate the anterior segment with the illuminating light source (48) in a state where the projection of the ring target (R1, R2) by the ring target projecting optical system (45) is restricted and perform automatic alignment on the anterior segment of the examinee's eye (E) where the projection of the ring target (R1, R2) is restricted, and further measure the eye refractive power of the examinee's eye (E) during wear of eyeglasses by the measuring optical system (10) after the automatic alignment is completed.

8. The eye refractive power measurement apparatus (1) according to any of claims 1 and 7, comprising a detector configured to detect whether or not an eyeglass lens is worn for the examinee's eye (E), wherein the control means controls the projection of the ring target (R1, R2) by the ring target projecting optical system (45) on the basis of a detection signal from the detector.

9. An eye refractive power measurement apparatus (1) according to claim 1, comprising:
a detector configured to detect whether or not an eyeglass lens is worn for the examinee's eye (E); and
mode switching means configured to switch between a first measurement mode for measuring the eye refractive power in an eyeglasses not worn state and a second measurement mode for measuring the eye refractive power in an eyeglasses worn state, on the basis of a detection signal from the detector.

10. The eye refractive power measurement apparatus (1) according to claim 9, comprising control means configured to determine a measurement result by the measuring optical system (10) in accordance with the measurement mode and output the measurement result.

## Patentansprüche

1. Eine Augenbrechkraftmessvorrichtung (1), die folgende Merkmale aufweist:
ein optisches Messsystem (10) zum Projizieren von Messlicht auf den Hintergrund eines Auges einer untersuchten Person (E), zum Empfangen von reflektiertem Licht von demselben und zum entsprechenden objektiven Messen der Augenbrechkraft des Auges der untersuchten Person (E);
eine Beleuchtungslichtquelle (48) zum Beleuchten des vorderen Segments des Auges der untersuchten Person (E) in einem Brille-wird-getragen-Zustand, wobei die Beleuchtungslichtquelle (48) in eine Position mit einer Neigung von 30° oder mehr in Bezug auf eine optische Messachse (L1) des optischen Messsystems (10) gebracht wird, die sich in Richtung des Auges der untersuchten Person (E) erstreckt;
ein optisches Betrachtungssystem (50), das dazu konfiguriert ist, das vordere Segment des Auges der untersuchten Person (E) in dem Brille-wird-getragen-Zustand zu betrachten, wobei das vordere Segment durch die Beleuchtungslichtquelle (48) beleuchtet wird; und
ein optisches Ringzielprojektionssystem (45), das dazu konfiguriert ist, ein Ringziel (R1, R2) in einen zentralen Bereich der Hornhaut des Auges der untersuchten Person (E) zu projizieren, wobei die Beleuchtungslichtquelle (48) in eine Position gebracht wird, die weiter von der optischen Messachse (L1) als das optische Ringzielprojektionssystem (45) entfernt ist, wobei
die Augenbrechkraftmessvorrichtung (1) in der Lage ist, die Augenbrechkraft des Auges der untersuchten Person (E) in dem Brille-wird-getragen-Zustand objektiv zu messen.

2. Die Augenbrechkraftmessvorrichtung (1) gemäß Anspruch 1, die folgende Merkmale aufweist:
eine Modusumschalteinrichtung, die dazu konfiguriert ist, zwischen einem ersten Messmodus zum Messen der Augenbrechkraft in einem Brille-wird-nicht-getragen-Zustand und einem zweiten Messmodus zum Messen der Augenbrechkraft in dem Brille-wird-getragen-Zustand umzuschalten; und
eine Steuereinrichtung (80), die dazu konfiguriert ist, das optische Ringzielprojektionssystem (45) gemäß dem Messmodus, in den von der Modusumschalteinrichtung umgeschaltet wird, zu steuern.

3. Die Augenbrechkraftmessvorrichtung (1) gemäß Anspruch 2, die einen Detektor aufweist, der dazu konfiguriert ist, zu erfassen, ob für das Auge der untersuchten Person (E) ein Brillenglas getragen wird oder nicht, wobei die Augenbrechkraftmessvorrichtung (1) dazu konfiguriert ist, die Messmodi auf der Basis eines Erfassungssignals von dem Detektor umzuschalten.

4. Die Augenbrechkraftmessvorrichtung (1) gemäß einem der Ansprüche 1 bis 3, die eine Ausrichtungsmodusumschalteinrichtung aufweist, die dazu konfiguriert ist, zwischen einem ersten Ausrichtungsmodus zum Ausrichten einer Messeinheit, die das optische Messsystem (10) umfasst, mit dem vorderen Segment des Auges der untersuchten Person (E) in dem Brille-wird-nicht-getragen-Zustand und einem zweiten Ausrichtungsmodus zum Ausrichten der Messeinheit mit dem vorderen Segment des Auges der untersuchten Person (E) in dem Brille-wird-getragen-Zustand umzuschalten, wobei
die Augenbrechkraftmessvorrichtung (1) dazu konfiguriert ist,
einen Ausrichtungszustand unter Verwendung des Ringziels (R1, R2) zu erfassen, das auf das Einstellen des ersten Ausrichtungsmodus hin von dem optischen Ringzielprojektionssystem (45) projiziert wird, und
einen Ausrichtungszustand unter Verwendung eines Merkmalsabschnitts des vorderen Segments des Auges der untersuchten Person (E) zu erfassen, das auf das Einstellen des zweiten Ausrichtungsmodus hin von der Beleuchtungslichtquelle (48) beleuchtet wird.

5. Die Augenbrechkraftmessvorrichtung (1) gemäß einem der Ansprüche 1 bis **4,** wobei die Beleuchtungslichtquelle (48) bei Messung des Hornhautdurchmessers oder Pupillendurchmessers des Auges der untersuchten Person (E) auch als Beleuchtung für das vordere Segment dient.

6. Die Augenbrechkraftmessvorrichtung (1) gemäß einem der Ansprüche 1 bis 5, wobei die Beleuchtungslichtquelle (48) eine Punktlichtquelle ist.

7. Die Augenbrechkraftmessvorrichtung (1) gemäß Anspruch 1, die eine Steuereinrichtung aufweist, die dazu konfiguriert ist, das vordere Segment mit der Beleuchtungslichtquelle (48) in einem Zustand zu beleuchten, in dem die Projektion des Ringziels (R1, R2) durch das optische Ringzielprojektionssystem (45) eingeschränkt ist, und eine automatische Ausrichtung des vorderen Segments des Auges der untersuchten Person (E) durchzuführen, wo die Projektion des Ringziels (R1, R2) eingeschränkt ist, und die Augenbrechkraft des Auges der untersuchten Person (E) während des Tragens einer Brille durch das optische Messsystem (10) weiter zu messen, nachdem die automatische Ausrichtung abgeschlossen ist.

8. Die Augenbrechkraftmessvorrichtung (1) gemäß einem der Ansprüche 1 und 7, die einen Detektor aufweist, der dazu konfiguriert ist, zu erfassen, ob für das Auge der untersuchten Person (E) ein Brillenglas getragen wird oder nicht, wobei die Steuereinrichtung die Projektion des Ringziels (R1, R2) durch das optische Ringzielprojektionssystem (45) auf der Basis eines Erfassungssignals von dem Detektor steuert.

9. Eine Augenbrechkraftmessvorrichtung (1) gemäß Anspruch 1, die folgende Merkmale aufweist:
einen Detektor, der dazu konfiguriert ist, zu erfassen, ob für das Auge der untersuchten Person (E) ein Brillenglas getragen wird oder nicht; und
eine Modusumschalteinrichtung, die dazu konfiguriert ist, zwischen einem ersten Messmodus zum Messen der Augenbrechkraft in einem Brille-wird-nicht-getragen-Zustand und einem zweiten Messmodus zum Messen der Augenbrechkraft in einem Brille-wird-getragen-Zustand auf der Basis eines Erfassungssignals von dem Detektor umzuschalten.

10. Die Augenbrechkraftmessvorrichtung (1) gemäß Anspruch 9, die eine Steuereinrichtung aufweist, die dazu konfiguriert ist, ein Messergebnis des optischen Messsystems (10) gemäß dem Messmodus zu bestimmen und das Messergebnis auszugeben.

## Revendications

1. Appareil (1) de mesure de puissance réfractive oculaire comprenant :
un système optique de mesure (10) destiné à projeter de la lumière de mesure sur le fond d'œil d'un œil (E) d'une personne examinée, à recevoir la lumière réfléchie depuis celui-ci et à mesurer en conséquence de manière objective la puissance réfractive de l'œil de l'œil (E) de la personne examinée ;
une source de lumière d'éclairage (48) destinée à éclairer le segment antérieur de l'œil (E) de la personne examinée dans un état de port de lunettes, la source de lumière d'éclairage (48) étant placée à une position inclinée de 30° ou plus par rapport à un axe optique de mesure (L1) du système optique de mesure (10), qui s'étend vers l'œil (E) de la personne examinée ;
un système optique d'observation (50) configuré pour observer le segment antérieur de l'œil (E) de la personne examinée dans l'état de port de lunettes, le segment antérieur étant éclairé par la source de lumière d'éclairage (48) ; et
un système optique de projection (45) de cible annulaire configuré pour projeter une cible annulaire (R1, R2) dans une zone centrale de la cornée de l'œil (E) de la personne examinée, dans lequel la source de lumière d'éclairage (48) est placée à une position plus éloignée de l'axe optique de mesure (L1) que le système optique de projection (45) de cible annulaire, dans lequel
l'appareil (1) de mesure de puissance réfractive oculaire est apte à mesurer de manière objective la puissance réfractive oculaire de l'œil (E) de la personne examinée dans l'état de port de lunettes.

2. Appareil (1) de mesure de puissance réfractive oculaire selon la revendication 1, comprenant :
un moyen de commutation de mode configuré pour alterner entre un premier mode de mesure destiné à mesurer la puissance réfractive oculaire dans un état de non-port de lunettes et un second mode de mesure destiné à mesurer la puissance réfractive oculaire dans l'état de port de lunettes ; et
un moyen de commande (80) configuré pour commander le système optique de projection (45) de cible annulaire conformément au mode de mesure commuté par le moyen de commutation de mode.

3. Appareil (1) de mesure de puissance réfractive oculaire selon la revendication 2, comprenant un détecteur configuré pour détecter si un verre de lunettes est porté ou non pour l'œil (E) de la personne examinée, dans lequel l'appareil (1) de mesure de puissance réfractive oculaire est configuré pour commuter les modes de mesure sur la base d'un signal de détection provenant du détecteur.

4. Appareil (1) de mesure de puissance réfractive oculaire selon l'une quelconque des revendications 1 à 3, comprenant un moyen de commutation de mode d'alignement configuré pour alterner entre un premier mode d'alignement destiné à aligner une unité de mesure incluant le système optique de mesure (10) avec le segment antérieur de l'œil (E) de la personne examinée dans l'état de non-port de lunettes et un second mode d'alignement destiné à aligner l'unité de mesure avec le segment antérieur de l'œil (E) de la personne examinée dans l'état de port de lunettes, dans lequel
l'appareil (1) de mesure de puissance réfractive oculaire est configuré pour
détecter un état d'alignement à l'aide de la cible annulaire (R1, R2) projetée par le système optique de projection (45) de cible annulaire lorsque le premier mode d'alignement est réglé, et
détecter un état d'alignement à l'aide d'une partie caractéristique du segment antérieur de l'œil (E) de la personne examinée éclairé par la source de lumière d'éclairage (48) lorsque le second mode d'alignement est réglé.

5. Appareil (1) de mesure de puissance réfractive oculaire selon l'une quelconque des revendications 1 à 4, dans lequel la source de lumière d'éclairage (48) sert également d'éclairage de segment antérieur lors de la mesure du diamètre de la cornée ou du diamètre de la pupille de l'œil (E) de la personne examinée.

6. Appareil (1) de mesure de puissance réfractive oculaire selon l'une quelconque des revendications 1 à 5, dans lequel la source de lumière d'éclairage (48) est une source de lumière ponctuelle.

7. Appareil (1) de mesure de puissance réfractive oculaire selon la revendication 1, comprenant un moyen de commande configuré pour éclairer le segment antérieur avec la source de lumière d'éclairage (48) dans un état où la projection de la cible annulaire (R1, R2) par le système optique de projection (45) de cible annulaire est limitée et effectuer un alignement automatique sur le segment antérieur de l'œil (E) de la personne examinée où la projection de la cible annulaire (R1, R2) est limitée, et mesurer en outre la puissance réfractive oculaire de l'œil (E) de la personne examinée pendant le port de lunettes par le système optique de mesure (10) après la réalisation de l'alignement automatique.

8. Appareil (1) de mesure de puissance réfractive oculaire selon l'une quelconque des revendications 1 et 7, comprenant un détecteur configuré pour détecter si un verre de lunettes est porté ou non pour l'œil (E) de la personne examinée, dans lequel le moyen de commande commande la projection de la cible annulaire (R1, R2) par le système optique de projection (45) de cible annulaire sur la base d'un signal de détection provenant du détecteur.

9. Appareil (1) de mesure de puissance réfractive oculaire selon la revendication 1, comprenant :
un détecteur configuré pour détecter si un verre de lunettes est porté ou non pour l'œil (E) de la personne examinée ; et
un moyen de commutation de mode configuré pour alterner entre un premier mode de mesure destiné à mesurer la puissance réfractive oculaire dans un état de non-port de lunettes et un second mode de mesure destiné à mesurer la puissance réfractive oculaire dans un état de port de lunettes, sur la base d'un signal de détection provenant du détecteur.

10. Appareil (1) de mesure de puissance réfractive oculaire selon la revendication 9, comprenant un moyen de commande configuré pour déterminer un résultat de mesure par le système optique de mesure (10) conformément au mode de mesure et délivrer en sortie le résultat de mesure.
